(19) Europäisches Patentamt
European Patent Office
Office européen des brevets

(11) Veröffentlichungsnummer: **0 098 566 B1**

# (12) EUROPÄISCHE PATENTSCHRIFT

(45) Veröffentlichungstag der Patentschrift: **21.08.91**

(51) Int. Cl.$^5$: **A61K 9/10**

(21) Anmeldenummer: **83106510.7**

(22) Anmeldetag: **04.07.83**

Die Akte enthält technische Angaben, die nach dem Eingang der Anmeldung eingereicht wurden und die nicht in dieser Patentschrift enthalten sind.

## (54) Kortikoidhaltige Zubereitung zur topischen Applikation.

(30) Priorität: **07.07.82 DE 3225848**

(43) Veröffentlichungstag der Anmeldung:
**18.01.84 Patentblatt 84/03**

(45) Bekanntmachung des Hinweises auf die Patenterteilung:
**21.08.91 Patentblatt 91/34**

(84) Benannte Vertragsstaaten:
**AT BE CH DE FR GB IT LI LU NL SE**

(56) Entgegenhaltungen:
**EP-A- 0 042 827**
**EP-A- 0 065 929**
**DE-A- 1 949 740**
**DE-A- 2 514 873**

**DERWENT JAPANESE PATENTS REPORT, Band R, Nr. 47, Januar 15, 1971, Artikel Nr. 87444R, LONDON, (GB).**

**CHEMICL ABSTRACTS, Band 94, Nr. 18, Mai, 1981 Seiten 378-9, Zusammenfassung, Nr. 145 389j. COLUMBUS, OHIO, (US).**

**SEIFEN-OLE-FETTE-WACHSE, Band 110, Nr. 10, Juni 28, 1984, Seiten 287-294, AUGSBURG, (DE). B. ZIOLKOWSKY: "Emulsionen in der Kosmetik: Theorie und Praxis".**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Stindl, Wolfgang**
**Kleinhöfleiner Hauptstrasse 24**
**A-7000 Eisenstadt(AT)**
Erfinder: **Zimmermann, Ingfried, Dr.**
**Gollanczstrasse 28c**
**W-1000 Berlin 28(DE)**
Erfinder: **Reckers, Renate, Dr.**
**Kufsteiner Strasse 2**
**W-1000 Berlin 62(DE)**
Erfinder: **Wendt, Hans, Dr.**
**Ernst-Ring-Strasse 14**
**W-1000 Berlin 38(DE)**
Erfinder: **Arndt, Rainold, Dr.**
**Undinestrasse 8**
**W-1000 Berlin 45(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft eine kortikoidhaltige Zubereitung zur topischen Applikation enthaltend 0,005 bis 2 Gewichtsprozent eines antiinflammatorisch wirksamen Kortikoids der allgemeinen Formel I

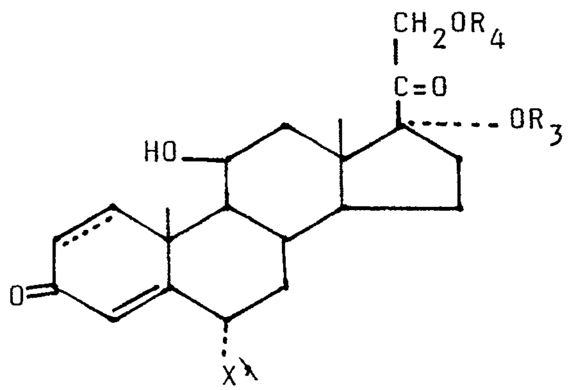

(I),

worin

..... eine Einfachbindung oder eine Doppelbindung,

X ein Wasserstoffatom oder eine Methylgruppe bedeutet, und worin

$R_3$ und $R_4$ gleich oder verschieden sind und Wasserstoff, 2 bis 6 Kohlenstoffatome enthaltende Alkanoylgruppen, Alkoxymethylgruppen oder Benzoylgruppen darstellen, gegebenenfalls zusätzliche hydrophile und/oder lipophile Wirkstoffe, sowie eine Fettphase, eine wässrige Phase, Emulgatoren, Konservierungsmittel sowie gegebenenfalls Duftstoffe, welche dadurch gekennzeichnet ist, daß sie das Kortikoid, die hydrophilen und/oder lipophilen Wirkstoffe, die Fettphase und wässrige Phase in Form einer feinstdispersen Mischung einer unverändert nebeneinander existierenden einen Öl/Wasser-Emulgator und Konservierungsmittel enthaltenden Öl/Wasser-Emulsion und einer einen Wasser/Öl-Emulgator enthaltenden Wasser/Öl-Emulsion enthält.

Kortikoide der allgemeinen Formel I, die sich zur Herstellung der erfindungsgemäßen kortikoidhaltigen Zubereitungen eignen, sind Hydrocortison, Prednisolon, 6α-Methylhydrocortison, 6α-Methylprednisolon und deren Ester wie zum Beispiel das Hydrocortison-21-acetat, das Hydrocortison-17-butyrat, das Hydrocortison-17-valerianat, das Prednisolon-21-acetat, das Prednisolon-17-valerianat, das 6α-Methylhydrocortison-21- acetat, das 6α-Methylprednisolon-21-acetat, das 6α-Methylhydrocortison-17-butyrat-21-acetat, das 6α-Methylprednisolon-21-acetat-17-propionat, das 6α-Methylhydrocortison- 17,21-dipropionat oder das 21-Acetoxy-17α-ethoxymethoxy- 11ß-hydroxy-1,4-pregnadien-3,20-dion.

Die erfindungsgemäße Zubereitung enthält 0,005 bis 2 und vorzugsweise 0,05 bis 0,5 Gewichtsprozent Kortikoid; die Konzentration desselben ist selbstverständlich von der relativen Wirksamkeit des Kortikoids abhängig.

Die Erfindung betrifft weiter ein Verfahren zur Herstellung derartiger kortikoidhaltigen Zubereitungen, das dadurch gekennzeichnet ist, daß man aus Fettphase, wässriger Phase, Öl/Wasser- Emulgator und Konservierungsmittel eine Öl/Wasser-Emulsion und aus Fettphase, wässriger Phase und Wasser/Öl-Emulgator eine Wasser/Öl-Emulsion herstellt und daß man die beiden Emulsionen unter Zusatz von hydrophilen und/oder lipophilen Wirkstoffen unter Vakuum bei einer Temperatur zwischen 20 und 40° C innig verrührt, so daß beide Emulsionstypen unverändert nebeneinander erhalten bleiben und darauf die erhaltene feinstdisperse Mischung mit Duftstoffen versetzt.

Mit Hilfe der die feinstdisperse Mischung der beiden Emulsionstypen enthaltenden Creme kann auf der Haut je nach ihrer Feuchtigkeit auf ihren unterschiedlichen Bereichen bzw. je nach Hauttypus ein entsprechender Öl/Wasser- oder Wasser/Öl-Hydrolipidfilm erzeugt werden.

Aus der EP-A 0042827 ist eine kortikoidhaltige Zubereitung in einer Wasser/Öl/Wasser-Emulsion vorbekannt. Diese eignet sich aber nicht dazu, die unterschiedlichen Bereiche der Haut je nach Bedarf mit einem Öl/Wasser- oder Wasser/Öl-Hydrolipidfilm zu versehen.

Aus der JP-B 7037292 ist ein Verfahren vorbekannt, bei dem eine Wasser/Öl-Emulsion mit einer Öl/Wasser-Emulsion gemischt wird. Beide Emulsionstypen sind aber so stark wasserhaltig, daß sie zur Herstellung einer kortikoidhaltigen Zubereitung ungeeignet sind.

Mit Hilfe des erfindungsgemäßen Verfahrens gelingt es, kortikoidhaltige Zubereitungen bereitzustellen, die neben einer Öl/Wasser-Emulsion zusätzlich noch eine Wasser/Öl-Emulsion enthalten.

Wenn nun die beiden Emulsionstypen bei Temperaturen zwischen 20 und 40° C, vorzugsweise bei 30° C unter Vakuum besonders schonend, dessen ungeachtet jedoch sehr intensiv, in einen Rührwerksbehälter vereinigt werden, so daß schließlich eine sehr feine Dispersion der beiden Emulsionen, deren Teilchengröße zwischen 2 und 50 $\mu$m, vorzugsweise zwischen 5 und 15 $\mu$m beträgt erzielt wird, dann bleiben die beiden Emulsionstypen unverändert nebeneinander in der hergestellten Creme erhalten. Dieses Emulsionssystem bleibt bei erheblicher Verdünnung, z. B. mit Flüssigphase, stabil. Es wird angenommen, daß für die Stabilität dieses Emulsionssystems die Teilchengröße ebenso wie das Zusammenwirken der Emulgatoren von Bedeutung ist. Wesentlich ist, daß bei der Herstellung der erfindungsgemäßen kortikoidhaltigen Zubereitung die zum Einsatz kommenden Emulsionen dispergiert und nicht homogenisiert werden.

Die Vereinigung der beiden Emulsionen erfolgt vorzugsweise unter einem Vakuum von 67 Pa bis 665 Pa (0.5 torr bis 50 torr). Die Rührgeschwindigkeit ist, wie dem Fachmann wohlbekannt ist, von dem verwendeten Rührwerk abhängig und muß in an sich bekannter Weise ermittelt werden.

Die Öl/Wasser-Emulsionen und die Wasser/Öl-Emulsionen können in konventioneller Weise unter Verwendung konventioneller Emulgatoren hergestellt werden (Kirk Othmer: Enzyclopedia of Chemical Technology, 3. Auflage, 1979; John Wiley & Sons; New York etc. Vol 8, Seite 900 bis 930 und Dr. Otto-Albrecht Neumüller: Römpps Chemie Lexikon 7. Auflage, 1973; Franck'sche Verlagshandlung Stuttgart, Seite 1009 bis 1013). Die für diese Emulsionen verwendeten Wachse, Emulgatoren und übrigen Zusätze sind die gleichen, wie man sie konventioneller Weise bei emulgierten Hautpflegemitteln verwendet (Dr. Otto-Albrecht Neumüller: Römpps Chemie Lexikon, 7. Auflage 1973; Franck'sche Verlagshandlung Stuttgart, Seite 1427 und 1428).

Eine in der erfindungsgemäßen kortikoidhaltigen Zubereitung eingesetzte Öl/Wasser-Emulsion kann aus hydrophilen und/oder lipophilen Wirkstoffen, Fettphase, Öl/Wasser-Emulgator, wässriger Phase und Konservierungsmittel bestehen.

Als hydrophile und/oder lipophile Wirkstoffe können Feuchthaltefaktoren (Hydrokomplexe), wie zum Beispiel Glycerin, Polyäthylenglykole oder Aminosäuregemische, Puroba-Oil (= Jojoba-Öl), Vitamine (vorzugsweise Vitamin A und E) Vitalkomplexe (wie zum Beispiel Placentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl/Wasser-Emulsion eignen sich Kohlenwasserstoffe wie zum Beispiel Vaseline, Paraffine oder Stearin, oder Wachse wie zum Beispiel Bienenwachs. Geeignete Öl/Wasser-Emulgatoren sind beispielsweise Stearylalkohol, Polyoxyäthylenstearate (wie zum Beispiel MYRJ(R)), Komplexemulgatoren (wie zum Beispiel Amphoterin(R)) und Sorbitanfettsäureester (wie zum Beispiel Span(R)) oder Carboxyvinylpolymerisate (wie zum Beispiel Carbopol(R)). Die wässrige Phase kann zusätzlich noch Puffersubstanzen, wie zum Beispiel das Dinatriumsalz der Äthylendiamin-N,N,N',N'-tetraessigsäure und Konservierungsmittel wie Chlorquinaldol, Parabene oder Benzalkoniumchlorid enthalten.

In der Öl/Wasser-Emulsion beträgt der Anteil der inneren Emulsion vorzugsweise 30 bis 49 Gew. %, die Teilchengröße der inneren Emulsion liegt vorzugsweise zwischen 1$\mu$m und 100$\mu$m. Bei der Dispergierung beider Phasen wird sie nochmals zerkleinert und liegt im fertigen Produkt unterhalb von 50$\mu$m.

Eine in der erfindungsgemäßen kortikoidhaltigen Zubereitung einsetzbare Wasser/Öl-Emulsion besteht wiederum aus hydrophilen und/oder lipophilen Wirkstoffen, wie sie auch in der Öl/Wasser-Emulsion verwendet werden, Fettphase, Wasser/Öl-Emulgator und wässriger Phase. Als ölige Phase oder Fettphase der Wasser/ Öl-Emulsion können Kohlenwasserstoffe, z.B. Paraffine und Vaseline, synthetische, pflanzliche und tierische Öle bzw. Wachse (wie zum Beispiel Olivenöl, Erdnußöl, feines Knochenöl, Mandelöl, Lanolin, Bienenwachs oder Sonnenblumenöl) eingesetzt werden, als wässrige Phase gereinigtes demineralisiertes Wasser und als Wasser/Öl-Emulgator Wollfett (=Lanolin), Fettalkohole wie zum Beispiel Cetylalkohol, Myristylalkohol, Stearylalkohol oder Cerylalkohol, Fettsäureester, wie zum Beispiel Bienenwachs (Cera alba) oder Wachsalkoholester oder Mischester (wie zum Beispiel Dehymuls(R)).

In der Wasser/Öl-Emulsion beträgt der Anteil der inneren Emulsion vorzugsweise 30 bis 49 Gew. %, die Teilchengröße der inneren Emulsion liegt vorzugsweise zwischen l$\mu$m und 100$\mu$m. Bei der Dispergierung beider Phasen wird sie nochmals zerkleinert und liegt im fertigen Produkt unterhalb von 50$\mu$m.

Das Mischungsverhältnis von Öl/Wasser-Emulsion und Wasser/Öl-Emulsion liegt vorzugsweise bei 20 bis 80 Gew. % und insbesondere bei 35 % bis 65 % Öl/Wasser-Emulsion.

Das feinstdisperse System wird zusätzlich mit dem mikronisierten Kortikoid (Korngröße vorzugsweise 1 bis 20 $\mu$m) und gegebenenfalls noch mit Duftstoffen, wie zum Beispiel diejenige der Crematest(R) -Serie versetzt und bis zur gleichmäßigen Verteilung derselben gerührt.

Die erfindungsgemäße kortikoidhaltige Zubereitung in Form einer Creme kann beispielsweise folgende Zusammensetzung haben:

| | | Toleranzen |
|---|---|---|
| Hydrocortison-21-acetat mikronisiert | 0,5 % | 0,1 - 1 % |
| (Teilchengröße bis µm) | | |
| Pur-oba-Oil | 5 % | 5 - 10 % |
| Cera-alba (Bienenwachs) | 1 % | 1 - 5 % |
| Dehymuls(R) | 1 % | 1 - 3 % |
| Stearylalkohol | 4 % | 4 - 8 % |
| Kohlenwasserstoffe | 30 % | 30 - 50 % |
| Carbopol(R) | 1 % | |
| MYRJ(R) | 3 % | 2 - 5 % |
| Dinatriumedetat | 1 % | |
| Chlorquinaldol | 1 % | |
| gereinigtes demineralisiertes Wasser | 52 % | 30 - 55 % |
| Parfumöl | 0,5 % | |

Die Angaben sind in Gewichtsprozent aufgeführt.

Die nachfolgenden Ausführungsbeispiele dienen zur Erläuterung des erfindungsgemäßen Verfahrens, welches mittels allen Vorrichtungen durchgeführt werden kann, die man konventioneller Weise zur Herstellung von Salben, Cremes etc. anwendet.

Beispiel 1

Herstellung der Ö1/Wasser-Emulsion.

10,00 g Dinatriumedetat und 10,00 g Chlorquinaldol werden in 300,00 g gereinigtem demineralisiertem Wasser gelöst und mit 10,00 g Carbopol(R) versetzt.

Diese Mischung wird unter kräftigem Rühren in eine Schmelze von 80,00 g Vaseline (DAB 8) - DAB ist die Abkürzung für das Deutsche Arzneibuch, amtliche Ausgabe, 8. Auflage 1978 - 40,00 g Stearylalkohol, 30,00 g MYRJ(R) und 50,00 g Puroba-Öl eingetragen. Man rührt die Mischung noch so lange, bis eine Emulsion mit einer Teilchengröße von 20-70 µm entsteht.

Herstellung der Wasser/Öl-Emulsion.

228,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch so lange, bis eine Emulsion mit einer Teilchengröße von 20-70 µm entsteht.

Herstellung einer Creme.

Die Wasser/Öl-Emulsion wird unter kräftigem Rühren unter einem Vakuum von 1330 Pa (10 torr) in die Öl/Wasser-Emulsion eingetragen. Man rührt noch solange, bis eine Dispersion mit einer Teilchengröße von 10 bis 50 µm entsteht, entfernt das Vakuum, setzt unter Rühren noch 5,000 g Hydrocortison-21-acetat - mikronisiert, Teilchengröße vorwiegend 1 bis 20µm, und 2,00 g eines Duftstoffes der Crematest(R) zu und rührt, bis sich beide Komponenten in der Salbengrundlage gleichmäßig verteilt haben.

Beispiel 2

Herstellung der Öl/Wasser-Emulsion.

10,00 g Dinatriumedetat und 10,00 g Chlorquinaldol werden in 300,00 g gereinigtem demineralisiertem Wasser gelöst und mit 10,00 g Carbopol(R) versetzt.

Diese Mischung wird unter kräftigem Rühren in eine Schmelze von 80,00 g Vaseline (DAB 8) - DAB ist die Abkürzung für das Deutsche Arzneibuch, amtliche Ausgabe, 8. Auflage 1978 - 40,00 g Stearylalkohol, 30,00 g MYRJ(R) und 50,00 g Puroba-Öl eingetragen. Man rührt die Mischung noch so lange, bis eine Emulsion mit einer Teilchengröße von 20-70 $\mu$m entsteht.

Herstellung der Wasser/Öl-Emulsion.

227,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch so lange, bis eine Emulsion mit einer Teilchengröße von 20-70 $\mu$m entsteht.

Herstellung einer Creme.

Die Wasser/Öl-Emulsion wird unter kräftigem Rühren unter einem Vakuum von 1330 Pa (10 torr) in die Öl/Wasser-Emulsion eingetragen. Man rührt noch so lange, bis eine Dispersion mit einer Teilchengröße von 10 bis 50 $\mu$m entsteht, entfernt das Vakuum, setzt unter Rühren noch 1,000 g 21-Acetoxy-11ß-hydroxy-6α-methyl-17α-propionyloxy-1,4-pregnadien-3,20-dion - mikronisiert, Teilchengröße vorwiegend 1 bis 20 $\mu$m, und 2,00 g eines Duftstoffes der Crematest(R) zu und rührt, bis sich beide Komponenten in der Salbengrundlage gleichmäßig verteilt haben.

Beispiel 3

Herstellung der Öl/Wasser-Emulsion.

10,00 g Dinatriumedetat und 10,00 g Chlorquinaldol werden in 300,00 g gereinigtem demineralisiertem Wasser gelöst und mit 10,00 g Carbopol(R) versetzt.

Diese Mischung wird unter kräftigem Rühren in eine Schmelze von 80,00 g Vaseline (DAB 8) - DAB ist die Abkürzung für das Deutsche Arzneibuch, amtliche Ausgabe, 8. Auflage 1978 - 40,00 g Stearylalkohol, 30,00 g MYRJ(R) und 50,00 g Puroba-Öl eingetragen. Man rührt die Mischung noch so lange, bis eine Emulsion mit einer Teilchengröße von 20-70 $\mu$m entsteht.

Herstellung der Wasser/Öl-Emulsion.

227,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch so lange, bis eine Emulsion mit einer Teilchengröße von 20-70 $\mu$m entsteht.

Herstellung einer Creme.

Die Wasser/Öl-Emulsion wird unter kräftigem Rühren unter einem Vakuum von 1330 Pa (10 torr) in die Öl/Wasser-Emulsion eingetragen. Man rührt noch so lange, bis eine Dispersion mit einer Teilchengröße von 10 bis 50 $\mu$m entsteht, entfernt das Vakuum, setzt unter Rühren noch 1,000 g 21-Acetoxy-17α-butyryloxy-11ß-hydroxy-6α-methyl-4-pregnen-3,20-dion - mikronisiert, Teilchengröße vorwiegend 1 bis 20 $\mu$m, und 2,00 g eines Duftstoffes der Crematest(R) zu und rührt, bis sich beide Komponenten in der Salbengrundlage gleichmäßig verteilt haben.

## Patentansprüche

1. Kortikoidhaltige Zubereitung zur topischen Applikation enthaltend 0,005 bis 2 Gewichtsprozent eines antiinflammatorisch wirksamen Kortikoids der allgemeinen Formel I

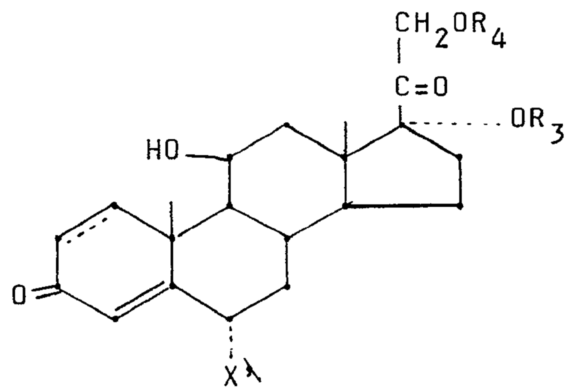

(I),

worin
..... eine Einfachbindung oder eine Doppelbindung,
X ein Wasserstoffatom oder eine Methylgruppe bedeutet, und worin
$R_3$ und $R_4$ gleich oder verschieden sind und Wasserstoff, 2 bis 6 Kohlenstoffatome enthaltende Alkanoylgruppen, Alkoxymethylgruppen oder Benzoylgruppen darstellen, gegebenenfalls zusätzliche hydrophile und/oder lipophile Wirkstoffe, sowie eine Fettphase, eine wässrige Phase, Emulgatoren, Konservierungsmittel sowie gegebenenfalls Duftstoffe, welche dadurch gekennzeichnet ist, daß sie das Kortikoid, die hydrophilen und/oder lipophilen Wirkstoffe, die Fettphase und wässrige Phase in Form einer feinstdispersen Mischung einer unverändert nebeneinander existierenden einen Öl/Wasser-Emulgator und Konservierungsmittel enthaltenden Öl/Wasser-Emulsion und einer einen Wasser/Öl-Emulgator enthaltenden Wasser/Öl-Emulsion enthält.

2. Kortikoidhaltige Zubereitung gemäß Patentanspruch 1 dadurch gekennzeichnet, daß die Teilchengröße der Emulsionen 2 bis 50 $\mu$m beträgt.

3. Verfahren zur Herstellung einer kortikoidhaltigen Zubereitung gemäß Patentanspruch 1 und 2, dadurch gekennzeizeichnet, daß man aus Fettphase, wässriger Phase, Öl/Wasser-Emulgator und Konservierungsmittel eine Öl/Wasser-Emulsion und aus Fettphase, wässriger Phase und Wasser/Öl-Emulgator eine Wasser/Öl-Emulsion herstellt und daß man die beiden Emulsionen unter Zusatz von hydrophilen und/oder lipophilen Wirkstoffen unter Vakuum bei einer Temperatur zwischen 20 und 40° C innig verrührt, so daß beide Emulsionstypen unverändert nebeneinander erhalten bleiben und darauf die erhaltene feinstdisperse Mischung mit dem mikronisierten Kortikoid und mit Duftstoffen versetzt.

## Claims

1. Corticoid-containing preparation for topical administration containing from 0.005 to 2 % by weight of a corticoid having anti-inflammatory activity of the general formula I

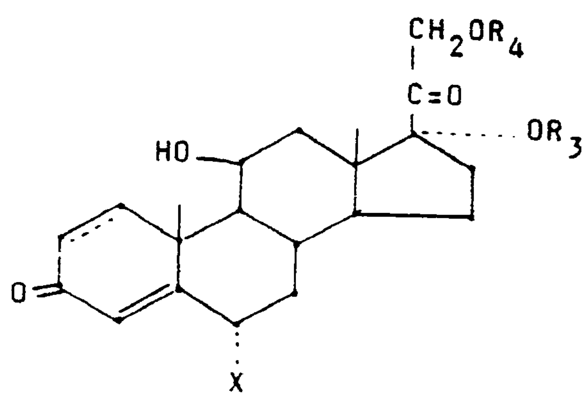

(I)

in which
---- represents a single bond or a double bond,

X represents a hydrogen atom or a methyl group and
$R_3$ and $R_4$ are the same or different and represent hydrogen, alkanoyl groups containing from 2 to 6 carbon atoms, alkoxymethyl groups or benzoyl groups, optionally additional hydrophilic and/or lipophilic active ingredients, and also a fatty phase, an aqueous phase, emulsifiers, preservatives as well as, optionally, perfumes, which is characterised in that it contains the corticoid, the hydrophilic and/or lipophilic active ingredients, the fatty phase and aqueous phase in the form of a very finely dispersed mixture, existing unchanged adjacent to each other, of an oil/water emulsion containing an oil/water emulsifier and preservative, and of a water/oil emulsion containing a water/oil emulsifier.

2. Corticoid preparation according to patent claim 1, characterised in that the particle size of the emulsions is from 2 to 50 $\mu$m.

3. Process for the manufacture of a corticoid-containing preparation according to patent claim 1 and 2, characterised in that an oil/water emulsion is prepared from fatty phase, aqueous phase, oil/water emulsifier and preservative, and a water/oil emulsion is prepared from fatty phase, aqueous phase and water/oil emulsifier, and that the two emulsions are intimately stirred in vacuo at a temperature of from 20 to 40° C with the addition of hydrophilic and/or lipophilic active ingredients, so that the two types of emulsion are retained unchanged adjacent to each other, and then the micronised corticoid and perfumes are added to the resulting very finely dispersed mixture.

**Revendications**

1. composition corticoïdique pour l'application locale, contenant de 0,005 à 2% en poids d'un corticoïde anti-inflammatoire répondant a la formule générale I :

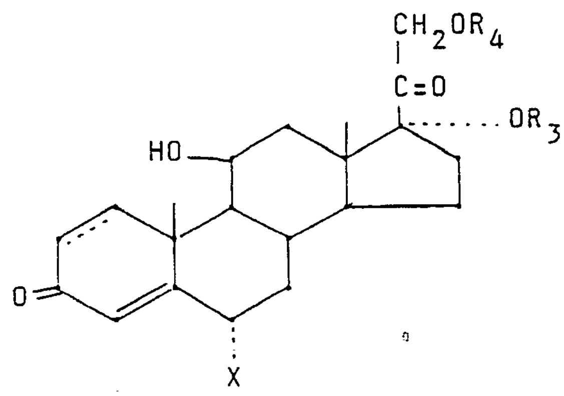

(I).

dans laquelle

........ représente une liaison simple ou une liaison double,
X représente un atome d'hydrogène ou un radical méthyle, et
$R_3$ et $R_4$ représentent chacun, indépendamment l'un de l'autre, l'hydrogène, un radical alcanoyle contenant de 2 à 6 atomes de carbone, un radical alcoxyméthyle ou un radical benzoyle,

éventuellement des substances actives hydrophiles et/ou lipophiles supplémentaires, ainsi qu'une phase grasse, une phase aqueuse, des émulsionnants, des conservateurs et éventuellement des parfums, composition caractérisée en ce qu'elle contient le corticoïde, les substances actives hydrophiles et/ou lipophiles, la phase grasse et la phase aqueuse sous la forme d'un mélange très finement dispersé constitué d'une émulsion LH ("huile-dans-l'eau") renfermant un émulsionnant LH et des conservateurs, et d'une émulsion HL ("eau-dans-l'huile") renfermant un émulsionnant HL, ces émulsions existant inaltérées côte-à-côte.

2. Composition corticoïdique selon la revendication 1 caractérisée en ce que la dimension des particules des émulsions est comprise entre 2 et 50 $\mu$m.

3. Procédé pour préparer une composition corticoïdique selon l'une des revendications 1 et 2, procédé caractérisé en ce qu'on prépare une émulsion LH à partir d'une phase grasse, d'une phase aqueuse,

d'un émulsionnant LH et d'un conservateur et une émulsion HL à partir d'une phase grasse, d'une phase aqueuse et d'un émulsionnant HL, et en ce qu'on mélange intimement les deux émulsions, tout en y ajoutant des substances actives hydrophiles et/ou lipophiles, sous pression réduite et à une température comprise entre 20 et 40° C, de telle façon que les deux types d'émulsion restent inaltérés côte-à-côte, puis on ajoute au mélange très finement dispersé obtenu le corticoïde micronisé et des parfums.